# EUROPEAN PATENT APPLICATION

(11) **EP 0 639 583 A1**
(43) Date of publication of application: **22.02.1995**
(21) Application number: 93710014.7
(22) Date of filing: 13.08.1993
(51) Int. Cl.: C07H 23/00, A61K 31/71, C12N 1/20, C12P 19/28

(54) **Antibiotics, called Salmycin A, B and C, a process for their preparation and their use as a pharmaceutical**

(71) Applicant: HOECHST AKTIENGESELLSCHAFT, D-65926 Frankfurt am Main (DE)
(72) Inventor: Vertesy, Laszlo, Dr., D-65817 Eppstein (DE); Aretz, Werner, Dr., D-61462 Königstein (DE); Fehlhaber, Hans-Wolfram, Dr., D-65510 Idstein (DE); Ganguli, Bimal Naresh, Dr., Bombay 400 071 (IN)

(57) **Abstract**

The antibiotics Salmycin A (C₄₁H₇₀FeN₇O₂₁, molecular weight 1052.9 g/mol), Salmycin B (C₄₁H₆₉FeN₆O₂₁, molecular weight 1037.88 g/mol) and Salmycin C (C₄₀H₆₇FeN₆O₂₁, molecular weight 1023.85 g/mol) are prepared by fermentation with Streptomyces violaceus 37290 (DSM 8286). The salmycines contain an amino-disaccharide component. The salmycines and their derivatives are used as a pharmaceutical.

## Description

The present invention relates to antibiotics, which are called Salmycin A, B and C, to a process for the preparation thereof from Streptomyces violaceus 37290, the variants thereof and to their use as a pharmaceutical.

Known iron containing antibiotics, socalled sideromycines, are ferrimycine [M. Bickel et al., Helv. Chim. Acta 43 (1960) 2105, albomycine [G. Benz et al., Angew. Chem. 94 (1982) 552], ferrocine [B. Katayama et al., J. Antibiotics 46 (1993) 65] and danomycine [P. Huber et al., Helv. Chim. Acta 69 (1986) 236]. These antibiotics show high in vivo activities. However, they exhibit severe disadvantages. The activity of ferrocine is restricted to Pseudomonas strains. Danomycine is not stable. Albomycine can be produced only in small yields.

Surprisingly, iron containing antibiotics were found, which do not exhibit the disadvantages of the known compounds and can be easily produced. The compounds according to the invention consist of an iron chelate and an amino-disaccharide-part. The structures of Salmycin A and B are represented by formulae Ia and Ib.

Accordingly, the subject of the instant invention are compounds of formula I 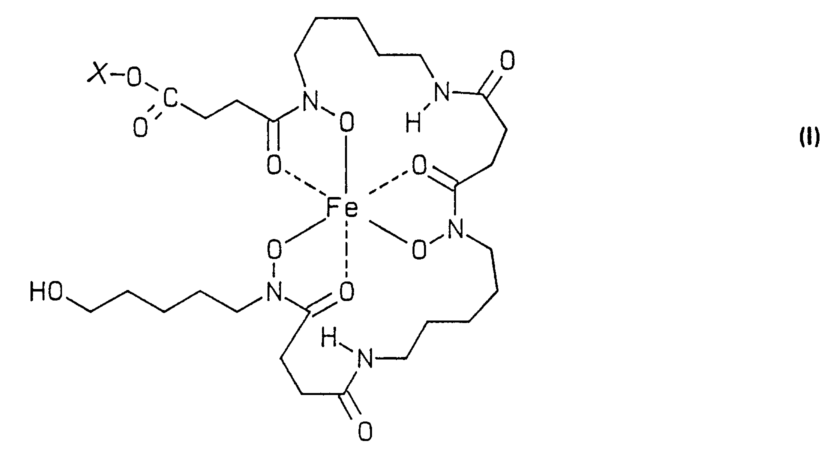
wherein X stands for -C₁₄H₂₄O₉NR, which is an amino-disaccharide group consisting of a hexose and an amino-heptose-unit; X contains a N-methyl group, but no carbamoyl or methoxy group; R denotes =NOH (a), =O (b); -OH and -H (c), -NH₂ and -H (d) or = N-O-R'(e); R' denotes phenyl or a branched or unbranched alkyl with 1 to 10 carbon atoms, preferably with 1 to 6 carbon atoms, in particular with 1 to 3 carbon atoms and a compound of the formula C₄₀H₆₇FeN₆O₂₁ (Salmycin C) which has a molecular weight of 1023,9 g/mol as well as the chemical equivalents and physiologically acceptable salts thereof. R is either double-bonded (cases a, b and e) or stands for a single bonded substituent and a H-atom (cases c and d).

Preferred are the compounds of formula I', 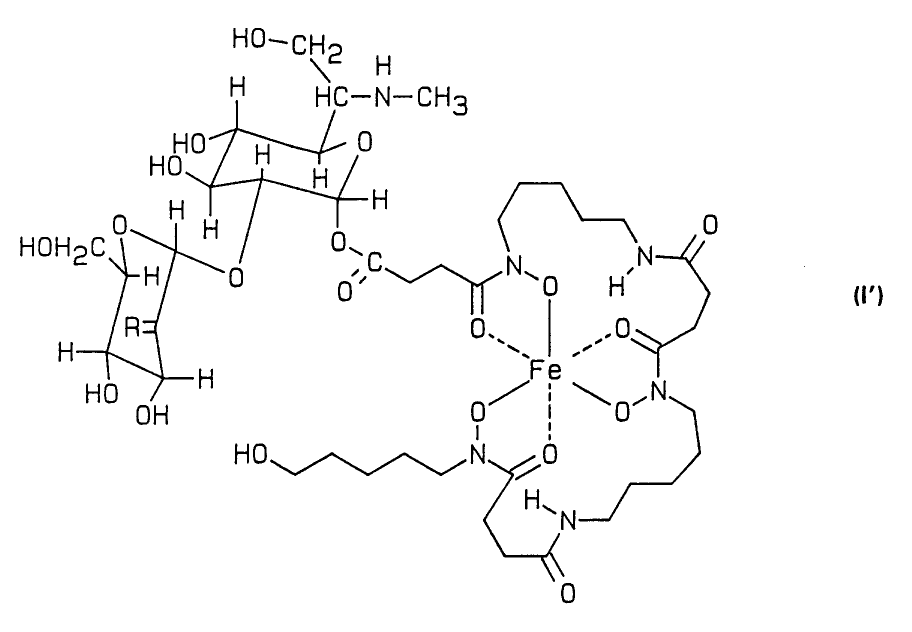
wherein X stands for -C₁₄H₂₄O₉NR, which is an amino-disaccharide group consisting of a hexose and an amino-heptose-unit; X contains a N-methyl group, but no carbamoyl or methoxy group; R denotes = NOH (a), = O (b);
-OH and -H (c), -NH₂ and -H (d) or =N-O-R'(e); R' denotes phenyl or a branched or unbranched alkyl with 1 to 10 carbon atoms, preferably with 1 to 6 carbon atoms, in particular with 1 to 3 carbon atoms as well as the chemical equivalents and physiologically acceptable salts thereof. R is either double-bonded (cases a, b and e) or stands for a single bonded substituent and a H-atom (cases c and d).

Salmycin A has a molecular weight of 1052,9 g/mol (molecular formula C₄₁H₇₀FeN₇O₂₁), Salmycin B has a molecular weight of 1037,9 g/mol (molecular formula C₄₁H₆₉FeN₆O₂₁). The structure of Salmycin C is not elucidated yet. The salmycines show a high antibacterial activity, in particular against gram-positive bacteria.

Since the activity mechanism of salmycines and their derivatives differs from that of conventional antibiotics, they are valuable pharmaceuticals, in particular in cases where resistances of gram-positive bacteria occur.

Hydrolysis of Salmycin A and B yields a chelate-component (siderophor), which is identical to danoxamin [P. Huber et al., Helv. Chim. Acta 69 (1986) 236], and a disaccharide-component, which is represented by X in formula I. The disaccharide-component of Salmycin B seems to consist of 6-methylamino-heptose and 2-keto-glucose. Salmycin A contains the oxime of 2-keto-glucose. Those disaccharide-components were not found in sideromycines yet. Accordingly, another subject of the instant invention are the disaccharidecomponents and their derivatives of formula II, wherein X has the same definition as in formula I. In formula II R denotes =N-OH (a), =O (b), -OH and -H (c), -NH₂ and -H (d), = N-O-R' (e); R' denotes phenyl or a branched or unbranched alkyl with 1 to 10 carbon atoms, preferably with 1 - 6 carbon atoms, in particular with 1 to 3 carbon atoms. R is either double-bonded (cases a, b and e) or stands for a single bonded substituent and a hydrogen-atom (cases c and d).

Preferred are the compounds of formula II',

X-H (II),

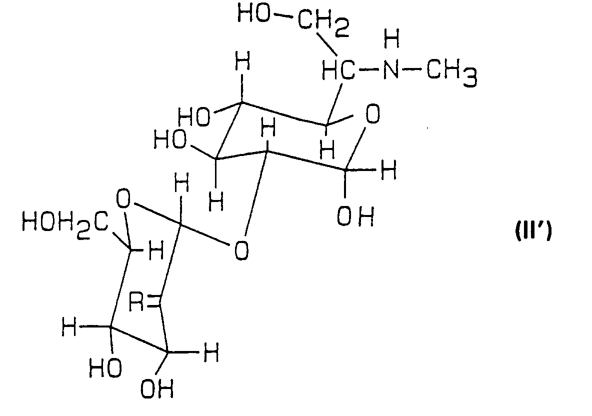

wherein R denotes =N-OH (a), =O (b), -OH and -H (c), -NH₂ and -H (d), =N-O-R' (e); R' denotes phenyl or a branched or unbranched alkyl with 1 to 10 carbon atoms, preferably with 1 - 6 carbon atoms, in particular with 1 to 3 carbon atoms. R is either double-bonded (cases a, b and e) or stands for a single bonded substituent and a H-atom (cases c and d).

Furthermore the present invention relates to a process for the preparation of Salmycines A, B and C by fermentation by means of the microorganism Streptomyces violaceus 37290 (deposited on May 13, 1993 with the "Deutsche Sammlung fur Mikroorganismen" according to the Treaty of Budapest with the number DSM 8286) or mutants or variants thereof; the Salmycines A, B and C are optionally transformed into their derivatives.

Mutants and variants can be generated for instance by radiation with UV- or X-rays or by use of chemical mutagenes, e.g. ethyl-methyl-sulphonate or 2-hydroxy-4-methoxy-benzophenone. These methods are state of the art.

The culture broth for the fermentation by Streptomyces violaceus 37290 contains a carbon and nitrogen source and the usual inorganic salts.

As carbon source for instance carbohydrates or sugar alcohols like glucose, lactose or mannitol and natural products like malt extracts, oils and fats can be used. Nitrogen sources are amino acids, peptides and proteins and their metabolites like peptones and tryptones; meat extracts; milled seeds like corn, wheat, beans, soybeans or cotton seeds; distillation residues of the alcohol production; meat powder or yeast extracts; ammonium salts or nitrates.

The culture broth can contain salts like chlorides, carbonates, sulfates or phosphates and salts of the alkali or earth alkali elements and trace elements like iron, zinc, cobalt or manganese.

The production of salmycines is particularly effective in a culture broth with 0.1 to 15 weight-% nutrients, preferably in the range of 0.1 to 3 weight-%. Nutrients like soybean flour, soybean oil or mannitol can be used. Fermentation is preferably performed under aerobic conditions. The culture fluid may be aerated (air or oxygen). It may be shaken or stirred. It is preferably a submerged fermentation. The temperature range for the fermentation may be 18 to 35°C, preferably 25 to 30°C, in particular 28 to 30°C. The pH-value of the culture medium should range between pH 6 and pH 8. A pH-value between pH 6.5 and pH 7.5 is preferred. Under these conditions salmycines are generally obtained within 1 to 5 days.

It is adavantageous to carry out the fermentation in several steps.
A mycelium with spores can be produced by cultivation on a fluid or solid nutrient medium like yeast-malt-agar. A preculture may be formed by inoculation of a nutrient fluid and incubation for approximately 80 to 400 h. A main culture can be formed by mixing a nutrient fluid with preculture fluid, e.g. in a ratio of 1:10 volume parts. The course of fermentation can be monitored by checking the pH-value of the culture medium, the volume of the mycelium or testing of the biological activity. Mycelium and culture medium contain the produced salmycines. The main part of the product compounds are usually found in the culture fluid. The work-up may be easier, if the mycelium is separated from the fluid. This can be achieved by filtration or centrifugation.

Salmycines A - C can be isolated from the culture fluid by methods of the state of the art like adsorption, ion exchange, precipitation, reversed osmosis or chromatography. Chromatographic materials like ion exchange resins, molecular sieves, adsorption resins or reversed phases can be used. A preferred procedure for the isolation of the salmycines is adsorption on a resin. The adsorbent is preferably a styrene and divinylbenzene copolymer in a bead form, having a macroreticular structure. The adsorbent is used either in a column or a batch process. After contacting the culture fluid with the adsorbent resin, for example by passing the culture fluid over a packed column, the resin loaded with product is washed with water. The products can be eluted from the resin for example with a mixture of water and an alcohol like isopropanol. The amount of the alcohol may be increased during elution (use of a gradient).

Ultrafiltration with semi-permeable and perm-selective membranes or films can be used for concentrating and desalting of the eluted sample. Acidic byproducts can be removed by treatment with an anion exchange resin (column or batch). After chromatography with molecular sieve as stationary phase a raw product is obtained, which is further separated, e.g. by reversed phase-chromatography, using C₁₈-silica gel or adsorption resin preferably. The procedure yields finally the solid products Salmycines A, B and C in high purity and in high yield. The procedure is very efficient, easily performed and works very economically.

Derivatives of the salmycines, which are represented by formulae Ic-e, are preferably prepared as follows.

### Hydroxy-derivative (formula Ic):

The carbonyl group of Salmycin B can be reduced with customary reducing agents or by cathodic reduction. The reaction is preferably carried out in aequeous solution at pH 7 with sodium borohydride (NaBH₄). The reduction yields two isomeric compounds. The products are separated and purified by chromatography, preferably by reversed phase chromatography with C₁₈-silica gel as stationary phase.

### Amino-derivative (formula Id):

The carbonyl group of Salmycin B can be transformed into an amino group by reductive amination. Preferably Salmycin B is treated in the cold with a dry solution of ammonia (maximum 17 weight-% NH₃ in dry methanol at room temperature) in methanol and equimolar amounts of sodium borohydride (NaBH₄) under stirring. The amino product is formed within a few minutes. Solvent and excess ammonia can be evaporated under reduced pressure. The product is purified by chromatography, preferably by reversed phase chromatography with C₁₈-silica gel as stationary phase. Two isomers are obtained.

### Oxime-ether of Salmycin B (formula Ie):

For the production of oxime-ethers of Salmycin B phenoxy- or alkoxy-ammonium chloride, preferably methoxy- (CH₃ONH₃Cl) or ethoxy-ammonium chloride (C₂H₅ONH₃Cl), and an aequeous solution of Salmycin B are mixed. The reaction is carried out preferably in a solution of pH 7 and at room temperature for several hours. The work-up of the solution comprises concentration and desalting. The product can be purified by chromatography, preferably by reversed phase chromatography with C₁₈-silica gel as stationary phase. The two possible oxime-ethers are obtained.

Another subject of the instant invention is a process for the production of the compounds of formula IIa - c by hydrolysis of the corresponding salmycin derivative. Hydrolysis may be performed preferably at pH 9.5 by adding a base like 0.1 M NaOH or KOH solution to an aequeous solution of the salmycin derivative at room temperature (approximately 2 h reaction time).

Salmycin A may also be formed by reaction of Salmycin B with hydroxylamine.

A further subject of the instant invention are pharmaceuticals, containing Salmycin A,B and/or C, disaccharide-components, their derivatives or physiologically tolerated salts of these substances (i.e. one or more compounds of formulae Ia-e and IIa-e and their physiologically tolerated salts). The pharmaceuticals can be prepared by mixing one or more of said compounds with one or more pharmacologically tolerated vehicles or diluents such as, for example, fillers, emulsifiers, lubricants, masking flavours, colorants or buffer substances, and converted into a suitable pharmaceutical form such as, for example, tablets, capsules or a suspension or solution suitable for parenteral administration.

Examples of vehicles or diluents which may be mentioned are tragacanth, lactose, talc, agar-agar, polyglycols, hydrocolloids, ethanol and water. Suitable and preferred for parenteral administration are suspension or solutions in water. It is also possible to administer the active substance as such, without vehicles or diluents, in a suitable form, e.g. in capsules.

### Examples

### Example 1

### Culturing of Streptomyces violaceus for the preparation of salmycines

### Composition of the culture medium:

| | |
|---|---|
| Starch | 10g/l |
| Casein | 1 g/l |
| Pepton | 1 g/l |
| Yeast extract | 1 g/l |
| Malt extract | 10 g/l |
| K₂HPO₄ | 0,5 g/l |
| Agar | 15 g/l |

The medium is sterilized at 121 °C for 1/2 hour, cooled to 45°C and poured into Petri dishes. The Petri dishes are inoculated with a suspension of spores of Streptomyces violaceus and incubated for 8 days at 28°C. Storage at 4°C.

1 cm² of an incubated Petri dish is used for inoculation of a preculture of 500 ml volume.

### Composition of the culture fluid (preculture):

| | |
|---|---|
| Glucose | 15 g/l |
| Soybean flour | 15 g/l |
| Cornsteep liquor | 5 ml/l |
| CaCO₃ | 2 g/l |
| NaCl | 5 g/l |
| pH | 7.2. |

It is incubated for 2 days at 28°C and shaken on a rotary shaker at 240 rpm. On a next stage 25 ml of the preculture fluid are used for inoculation of 50 ml nutrient fluid, filled in 500 ml Erlenmeyer flasks (main culture).

The composition of the main culture fluid is:

| | |
|---|---|
| Soybean flour | 20 g/l |
| Mannitol | 20 g/l |
| pH | 7.5. |

Incubation conditions are: 28°C, shaking on a rotary shaker at 240 rpm, 3-4 days duration. Finally a diffusion test shows a biological activity of 21 - 23 mm diameter of inhibition zone of Staphylococcus aureus 209 P.

### Example 2

### Fermentation

The fermentation is carried out in 12 l-vessels with 9 l culture fluid (identical to main culture fluid, example 1: 20 g/l soybean flour + 20 g/l mannitol, pH 7.5). 90 ml of the main culture (example 1) are used for inoculation. Incubation conditions are: 28°C, shaking at 500 rpm, aeration rate of 0.5 liter per minute, 2-3 days duration.

After the fermentation is terminated the culture filtrate is tested using the agar well method. The diameter of a zone of inhibition of Staphylococcus aureus 209 P is usually in the range of 22 - 27 mm.

### Example 3

### Isolation of raw product

180 l broth filtrate of cultures prepared according to example 2 are brought to pH 6.8 and are passed through a 17 l-column packed with an adsorbent resin. A styrene and divinylbenzene copolymer in a bead form like ^{®}Diaion HP 20 (Mitsubishi Chem. Ind., Japan) is preferably used. The column is washed with demineralized water and is eluted with a concentration gradient of 0 - 20 Vol.-% isopropanol. Fractions are collected and their biological activity is tested.

Fractions containing salmycines are combined (ca. 30 l overall). The solution is liberated from acidic components by passing over an anion exchange column (1 l of diethylaminoethyl-sepharose like DEAE-^{®}Sephadex Fast Flow from Pharmacia Fine Chemicals AB, Sweden), which was equilibrated with an ammonium acetate solution.

The sample solution is then concentrated by ultrafiltration using semi-permeable and perm-selective ^{®}Nadir UF-CA-1-membranes (Hoechst; Frankfurt, Germany). The solvent is evaporated under reduced pressure and the residue is freeze-dried. 38 g of raw product mixture results.

### Example 4

### Pre-separation of raw product mixture

35 g raw product mixture prepared according to example 3 are dissolved in distilled water and passed over a 3.5 l-column (11.3 cm inner diameter; 36 cm height), which is packed with an adsorbent like "MCI-Gel CHP20P" (Mitsubishi Kasei Corp., Tokyo, Japan), based on a styrene and divinylbenzene copolymer. The column is washed with water. The products are eluted with a concentration gradient of 0 - 10 vol.-% isopropanol. 1 l-fractions are collected. Fractions containing a mixture of Salmycin B and C and fractions mainly of Salmycin A are obtained. Concentration under reduced pressure and lyophilization give 3.2 g raw mixture of Salmycin B and C and 1.4 g crude Salmycin A.

### Example 5

### Purification of Salmycin B and C

The raw mixture of Salmycin B and C obtained according to example 4 is passed over a column of 10 cm inner diameter and 52 cm height, filled with molecular sieve, such as ^{®}Fractogel TSK HW-40F (E. Merck, Darmstadt, Germany), which is a hydrophilic vinyl polymer made for gel permeation chromatograpghy for fractionating low molecular compounds like dextrans in the molecular range of 100-7000 g/mol. A mixture of equal parts of water and methanol and 1 vol.-% acetic acid is used as solvent. 24 ml fractions are collected. Fractions, containing Salmycin B or C, are combined and concentrated under reduced pressure. Lyophilization gives 510 mg of raw product, containing 82 weight-% Salmycin B and 3 weight-% Salmycin C.

### Example 6

### Purification of Salmycin A

1.4 g crude Salmycin A are purified by use of molecular sieve like in example 5. The solvent consists of 4 parts water and 1 part ethanol. Fractions containing Salmycin A are combined. Concentration under reduced pressure and lyophilization give 240 mg crude Salmycin A with a content of 85 weight-%.

### Example 7

### Final preparation of Salmycin A

The crude Salmycin A from example 6 is dissolved in distilled water and purified by reversed phase chromatography. A column of 3.2 cm inner diameter and 25 cm height was packed with 200 ml octadecyl-silica gel like ^{®}Nukleosil 12 C₁₈ AB (Macherey & Nagel, Düren, Germany). As solvent a water-acetonitrile-mixture is used with a concentration gradient of 0 - 10 vol.-% acetonitrile. 25 ml-fractions are collected. Fractions with Salmycin A are combined. Concentration under reduced pressure and lyophilization give 130 mg product, containing 99 % Salmycin A by weight.

### Characterization of Salmycin A:

a) high resolution FAB-mass spectrometry:
   molecule ion peak of M + H⁺: 1053.4050 ± 0,0006 dalton.
   mass calculated for M + H⁺ of C₄₁H₇₀FeN₇O₂₁, mono-isotopic: 1053.4052 dalton.
b) ¹H-NMR: signals at 2.8 ppm in D₂O (N-methyl group), no further signals for methyl-protons.
   ¹³C-NMR of Desferri-Salmycin A in D₂O: signals (in ppm) at 25.0 (t), 25.9 (t), 28.3 (t), 28.4 (t), 29.7 (t), 30.5 (t), 30.7 (t), 31.5 (t), [33.3 (t)], 33.4 (t), 33.7 (t), 42.0 (t), 50,8 (t), 58,6 (t), 58,8 (t), 61,8 (d), 62,3 (d), 64,4 (t), 62-82 (several signals), 91.5 (d), 92.4 (d), 99.1 (d), 176.6 (s), 177.7 (s), 177.8 (s).
c) UV-vis of aqueous solution (phosphate buffer, pH 7.0):
   end absorption and broad absorption around 430 nm (log ε = 3.3).

### Example 8

### Final preparation of Salmycin B and C

The mixture of Salmycin B and C obtained according to example 5 is purified by reversed phase chromatography in a manner similar to example 7. The solvent consists of a mixture of water, 6 vol.-% acetonitrile and 0.1 vol.-% trifluoro acetic acid. 402 mg Salmycin B and 8 mg Salmycin C are obtained.

### Characterization of Salmycin B:

a) high resolution FAB-mass spectrometry:
   molecule ion peak of M + H⁺: 1038.3941 ± 0.0007 dalton.
   mass calculated for M + H⁺ of C₄₁H₆₉FeN₆O₂₁, mono-isotopic: 1038.3943 dalton.
   molecule ion peak of M + H⁺ +H₂O: 1056.406 ± 0.001 dalton.
   mass calculated for M + H⁺ +H₂O: 1056.4053 dalton.
b) ¹H-NMR: signals at 2.8 ppm in D₂O (N-methyl group), no further signals for methyl-protons.
c) UV-vis of aqueous solution (phosphate buffer, pH 7.0):
   end absorption and broad absorption around 427 nm (log ε = 3.3).

### Characterization of Salmycin C:

a) ESI-MS-spectrometry (electron spray ionisation):

| | |
|---|---|
| molecular ion peak M + H⁺: | 1024.4 dalton. |
| molecular ion peak M + H⁺ +H₂O: | 1042.4 dalton. |
| general formula: | C₄₀H₆₇FeN₆O₂₁. |

b) ¹H-NMR: signals at 2.8 ppm in D₂O (N-methyl group), no further signals for methyl-protons.
c) UV-vis of aqueous solution (phosphate buffer, pH 7.0):
end absorption and broad absorption around 430 nm (log ε = 3.3).

The salmycines show zones of inhibition with isolated resistant strains which are typical for sideromycines. The agar diffusion test shows antibacterial activity of the salmycines against Streptococcus strains, Staphylococcus strains and Methicillin-resistent strains. A determination of the minimal concentration for inhibition is for said species not possible. Salmycin A shows in contrast to Salmycin B and C always clear zones of inhibition. Chymotrypsin or β-lactamase do not react with the salmycines.

### Example 9

### Reaction of Salmycin B with hydroxylamine forming Salmycin A

100 mg Salmycin B are dissolved in 100 ml distilled water and mixed with 100 mg hydroxyl-amine-hydrochloride. The pH-value is adjusted to pH 4.5 with phosphate buffer. Reaction time is 3 hours. The formed Salmycin A is isolated as described in example 7. 87 mg Salmycin A resulted, having a purity of 98 weight-%.

### Example 10

### Preparation of Salmycin A-oxime-methylether

100 mg Salmycin B are dissolved in 100 ml phosphate buffer, pH 7, and mixed with 100 mg O-methylhydroxyl-ammonium chloride (E. Merck, Art.-Nr. 10 592). Reaction time is 5 hours. Two forms of Salmycin A-oxime-methylether (syn and anti) are formed. They are isolated as described in example 7. 41 mg of the syn-form and 44 mg of the anti-form are obtained.
The reaction is monitored with a HPLC-system (^{®}Nukleosil 7-C₁₈ AB; Macherey & Nagel, Düren, Germany) with a mixture of water/8.75 vol.-% acetonitrile/0,1 vol.-% trifluoro acetic acid as solvent.

### Example 11

### Reduction products of Salmycin B

104 mg Salmycin B are dissolved in potassium phosphate buffer, pH 7.0, and mixed with 2 mg sodium borohydride. The reaction mixture is left at room temperature. The course of the reaction is monitored by HPLC (column: ^{®}Nukleosil 7-C₁₈AB (Macherey & Nagel); solvent: water/10 vol.-% acetonitrile/0.1 vol.-% trifluoro acetic acid). The reaction is almost completed after 2 hours. The reaction mixture is separated by preparative HPLC (column: 80 ml ^{®}Nukleosil 10-C₁₈ AB (Macherey & Nagel); solvent: water/6 vol.-% acetonitrile/0.1 vol.-% trifluoro acetic acid).

Fractions are concentrated under reduced pressure and lyophilized to give 37 mg mannosyl-reduction product and 33 mg glucosyl-reduction product. These products appear as salts of trifluoro acetic acid with a purity of 97 and 95 weight-%, respectively.

Both compounds show molecular peaks M + H⁺ = 1040 dalton in FAB-MS-Spectra, which corresponds to the molecular formula C₄₁H₇₁FeN₆O₂₁.

### Example 12

### Reductive amination of Salmycin B

10 g of dry gaseous ammonia (NH₃) are passed in 100 ml dry methanol at -10° C. 100 mg Salmycin B dissolved in 10 ml dry methanol and 5 mg sodium borohydride (NaBH₄) suspended in 5 ml methanol are added to this solution. The reaction mixture is stirred for 10 minutes. Solvent and NH₃ are removed under reduced pressure. The residue is purified as described in example 11. Two isomeric amino compounds are obtained, which are represented by formula Id.

## Claims

1. Compounds of formula I, 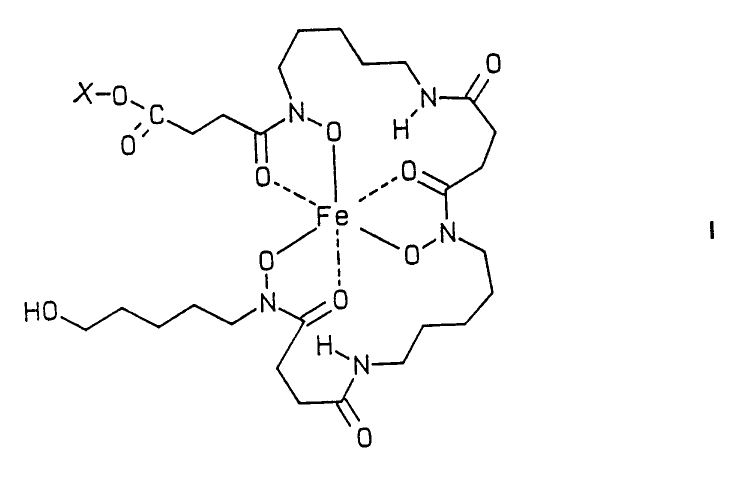 wherein X stands for -C₁₄H₂₄O₉NR, which is an amino-disaccharide group consisting of a hexose and an amino-heptose-unit; X contains a N-methyl group, but no carbamoyl or methoxy group; R denotes =NOH (a), =O (b); -OH and -H (c), -NH₂ and -H (d) or =N-O-R'(e);
R' denotes phenyl or a branched or unbranched alkyl with 1 to 10 carbon atoms,
and a compound of the formula C₄₀H₆₇FeN₆O₂₁ (Salmycin C), which has a molecular weight of 1023,9 g/mol;
as well as obvious chemical equivalents and physiologically acceptable salts thereof.

2. Compounds of formula I', 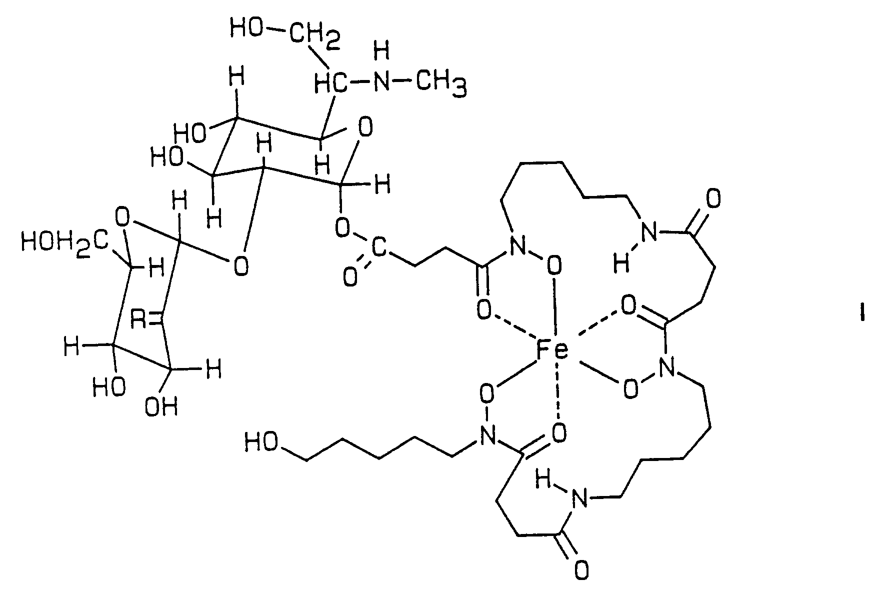 wherein R denotes =NOH (a), =O (b); -OH and -H (c), -NH₂ and -H (d) or =N-O-R'(e);
R' denotes phenyl or a branched or unbranched alkyl with 1 to 10 carbon atoms,
as well as obvious chemical equivalents and physiologically acceptable salts thereof.

3. Compounds of the formula X-H,
wherein X stands for an aminodisaccharide group consisting of a hexose and an amino-heptose-unit, which contains a N-methyl group, but no carbamoyl or methoxy group; R denotes =N-OH (a), =O (b), -OH and -H (c), -NH₂ and -H (d), =N-O-R' (e);
R' denotes phenyl or a branched or unbranched alkyl with 1 to 10 carbon atoms,
as well as obvious chemical equivalents and physiologically acceptable salts thereof.

4. Compounds of the formula II' 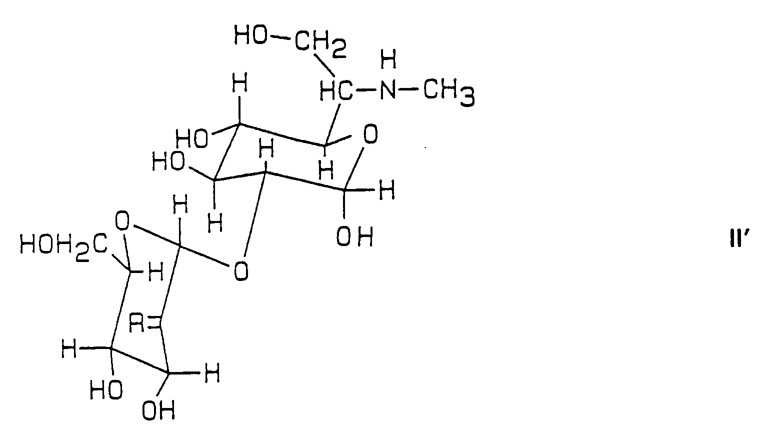 wherein R denotes =N-OH (a), =O (b), -OH and -H (c),
-NH₂ and -H (d), =N-O-R' (e);
R' denotes phenyl or a branched or unbranched alkyl with 1 to 10 carbon atoms,
as well as obvious chemical equivalents and physiologically acceptable salts thereof.

5. A process for the preparation of compounds as claimed in claim 1 and 2, which comprises cultivation of the microorganism Streptomyces violaceus 37290 (DSM 8286) under aerobic conditions in an aqueous nutrient medium, which contains carbon sources, nitrogen sources, inorganic nutrient sources and mineral salts and isolation of Salmycin A, B and C and optionally transforming the compounds obtained into their derivatives.

6. A process for the production of the compounds as claimed in claim 3 and 4, wherein a compound as claimed in claim 1 or 2 is hydrolized.

7. Compounds as claimed in claims 1 - 4 as a pharmaceutical.

8. The use of compounds as claimed in claims 1 - 4 for the preparation of pharmaceuticals having an antibiotic action.

9. Pharmaceuticals, which contain one or more compounds as claimed in claims 1 - 4, if appropriate together with customary auxiliaries and/or vehicles.

10. A process for the preparation of pharmaceuticals as claimed in claim 9, wherein one or more of the compounds as claimed in claims 1 - 4 are brought, optionally with customary auxiliaries and/or vehicles into a suitable form for administration.

11. Streptomyces violaceus 37290 (DSM 8286) as well as its variants or mutants.
